# EUROPEAN PATENT APPLICATION

(11) **EP 2 113 238 A1**
(43) Date of publication of application: **04.11.2009**
(21) Application number: 08252121.2
(22) Date of filing: 19.06.2008
(51) Int. Cl.: A61K 8/02, A61K 8/22, A61K 8/25, A61K 8/37, A61K 8/73, A61K 8/81, A61K 8/92, A61Q 11/00

(54) **PRODUCTS FOR WHITENING TEETH**

(30) Priority: 20.06.2007 US 821157; 21.06.2007 US 821217
(71) Applicant: McNeil-PPC, Inc., Skillman, NJ 08558 (US)
(72) Inventor: Georgiades, Constantine, East Brunswick, NJ 08816 (US); Mody, Seema, Montville, NJ 07045 (US); Soshinsky, Andre, Randolph, NJ 07869 (US); Zhang, Zhen (Jane), Basking Ridge, NJ 07920 (US)
(74) Representative: Kirsch, Susan Edith

(57) **Abstract**

This invention relates generally to films having barrier as well as controlled disintegration properties and, more particularly, to controlled water disintegrable films. The multi-layer film comprises a first layer, which contains a water insoluble polymer and a disintegration facilitator, and a second layer, comprising a tooth whitening active and a water soluble polymer.

## Description

### FIELD OF THE INVENTION

This invention relates generally to whitening products or devices comprising disintegrable film layers having controlled permeability and disintegration properties, and more particularly to devices having disintegrable films that provide controlled water permeability.

### BACKGROUND OF THE INVENTION

Many individuals desire whiter teeth and "brighter" smiles. Stained teeth are often considered dull and cosmetically unattractive. Unfortunately, without appropriate preventive or remedial measures, stained teeth are an inevitable consequence of the absorbent nature of dental material. Everyday activities such as smoking or other oral use of tobacco products, and eating, chewing or drinking certain foods and beverages (in particular coffee, tea and red wine), cause undesirable staining of surfaces of teeth. Staining can also result from microbial activity, including that associated with dental plaque. The chromogens or color causing substances in these materials become part of the pellicle layer and can permeate the enamel layer. Even with regular brushing and flossing, years of chromogen accumulation can impart noticeable tooth discoloration.

Recently, in an effort to solve the above problem, a number of home-based teeth whitening products have been introduced into the market.

Examples of recently introduced teeth whitening products include those incorporating strips or trays loaded with a teeth whitening composition, where the strip (or strip residue) or tray remains in the mouth after use, requiring subsequent removal.

Other teeth whitening products have also been introduced where the strip or carrier layer for the whitening composition dissolves in the mouth during use. These products, however, typically form the strip or carrier layer from water soluble polymers to achieve product dissolution during use.

Notwithstanding these attempts at improving the convenience of home-based teeth whitening products, there remains a need for teeth whitening products providing more controlled dissolution and disintegration properties.

In addressing these disadvantages, the inventors of the present invention have discovered that teeth whitening devices comprising a disintegrable film layer comprising select water insoluble polymers and a disintegration facilitator selected from the group consisting of a plasticizer, a water insoluble particulate or mixtures thereof provide protective properties as well as provide improved water permeability and disintegration control properties.

Therefore, an aspect of the present invention is to provide teeth whitening products comprising a disintegrable film layer having controlled (or an extended type or prolonged) disintegration and/or dissolution properties in aqueous environments.

Still one other aspect of the present invention is to provide teeth whitening products or devices that are dry to the touch during handling, yet erode in the oral cavity after application onto the teeth.

Still yet one other aspect of the present invention is to provide disintegrable film products for delivering teeth whitening actives wherein the film disintegrates within 60 minutes, optionally within 45 minutes, optionally, within 30 minutes or, optionally, within 15 minutes in an aqueous environment.

### SUMMARY OF THE INVENTION

In one embodiment, the teeth whitening device of the present invention comprises a first or disintegrable layer of a bi-layer film where the second layer comprises a teeth whitening active and a water soluble polymer film layer such as that described in US patents 6,596,298 to Leung et al. and 6,419,903 to Xu et al., both of which are herein incorporated by reference in their entirety. The bilayer device is then applied to the teeth, and allowed to erode over time in the presence of oral fluids or other aqueous media.

In another embodiment, the present invention relates to multi-layer teeth whitening device, comprising
i.) a first layer comprising:
   a.) at least one water insoluble polymer; and
   b.) at least one disintegration facilitator selected from the group consisting of water insoluble particulates, plasticizers and mixtures thereof; and
ii.) a second layer comprising:
   a.) at least one tooth whitening active; and
   b.) at least one water soluble polymer
   wherein the device is erodible in an aqueous environment.

Similarly, the disintegrable film of the present invention may be incorporated in multi-layer films and used as above to achieve the benefits of the present invention.

Methods of using the above film compositions are also disclosed.

### BRIEF DESCRIPTION OF THE DRAWINGS

The detailed description will be better understood in conjunction with the accompanying drawings, wherein like reference characters represent like elements, as follows:
FIGURE 1 is a perspective view of one exemplary disintegrable film product of the current invention;
FIGURE 2 is a perspective view of another exemplary disintegrable film product of the current invention, where the disintegrable film of the present invention forms the first or disintegrable layer of a bi-layer film;
FIGURE 3 is a perspective view of a disintegrable film product similar to that of FIGURE 2, but where the disintegrable film of the present invention is not of uniform thickness;
FIGURE 4 is a perspective view of a disintegrable film product similar to that of FIGURE 3, but where the disintegrable film of the present invention does not fully cover the second layer of a bi-layer film;
FIGURE 5 is a plan view of an exemplary film product having an exemplary positioning feature in accordance with the principles of the present invention;
FIGURE 6 is a plan view of a film product similar to that of FIGURE 5, but with a different exemplary positioning feature;
FIGURE 7 is a plan view of a film product similar to that of FIGURE 5, but with a different exemplary positioning feature;
FIGURE 8 is a plan view of a film product similar to that of FIGURE 5, but with a different exemplary positioning feature;
FIGURE 9 is a plan view of another exemplary film product having an exemplary positioning feature similar to that of FIGURE 7; and
FIGURE 10 is a perspective view of yet another exemplary film product, with another type of exemplary positioning feature.

### DETAILED DESCRIPTION OF THE PRESENT INVENTION

The disintegrable film compositions of the present invention can comprise, consist of, or consist essentially of the essential elements and limitations of the invention described herein, as well any of the additional or optional ingredients, components, or limitations described herein.

All percentages, parts and ratios are based upon the total weight of the wet film layer composition of the present invention, unless otherwise specified. All such weights as they pertain to the listed ingredients are based on the active level and, therefore, do not include carriers or by-products that may be included in commercially available materials, unless otherwise specified.

The term "safe and effective amount" as used herein means an amount of a compound or composition such as a topical or system active sufficient to significantly induce a positive benefit, for example, a teeth whitening, but low enough to avoid serious side effects, i.e., to provide a reasonable benefit to risk ratio, within the scope of sound judgment of the skilled artisan.

The term "dry", as used herein, means having a water content equal to or less than about 15%, optionally equal to or less than about 10%, optionally equal to or less than about 5%.

The term "adhesive" as used herein, means any material or composition that is capable of sticking to the site of topical application or administration and includes, but is no limited to, pressure-sensitive adhesive (adheres upon application of pressure), moistenable adhesives (adheres in the presence of water) and tacky or sticky type adhesives (adheres upon immediate contact with a surface).

The terms "erode" or "erodible" as used herein, mean a "wearing away" of the teeth whitening device as a result of the combination of dissolution (or dispersability) and disintegration processes. Specifically, the overall mechanism by which teeth whitening product of the invention is removed from the oral cavity after application to the teeth must include a disintegration component. Said another way, the teeth whitening product of the invention "erodes" only where some portion of the product disintegrates in the oral fluids. For example, a multi-layer teeth whitening product of the present invention is said to "erode" away in the oral cavity as used herein only where at least one of the layers disintegrates and does not dissolve in the fluids of the oral cavity.

Optionally, the film compositions of the present invention are clear. The term "clear" as defined herein ranges from transparent to translucent as observed with the naked eye.

The film compositions of the present invention, including the essential and optional components thereof, are described in detail hereinafter.

### The Water Insoluble Polymer

The disintegrable film compositions of the present invention comprise water insoluble polymers. Suitable water insoluble polymers include, but are not limited to, hydrogenated caster oil; polyvinyl chloride; shellac; cellulose derivatives such as cellulose or ethylcellulose; waxes. In certain embodiments, the water-insoluble polymer is brittle once the solvent is evaporated off by drying.

Examples of the waxes suitable for use herein include, but are not limited to, paraffin, carnauba wax, candelilla wax, sugarcane wax, beeswax, cetyl esters wax, montan wax, glycowax, castor wax, spermaceti wax, shellac wax, microcrystalline wax and mixtures thereof.

In certain embodiments, the water insoluble polymer has a molecular weight of from about 300 to about 700, optionally from about 390 to about 480 daltons.

Mixtures of any of the above ingredients can also be used.

In certain embodiment, the water insoluble polymer can be shellac. A suitable marketed shellac is sold under the name Pharmaceutical Glaze and supplied by Mantrose Haeser Co., Attleboro, MA.

The water insoluble polymer is present at a concentration of from about 5% to about 80%, optionally, from about 10% to about 50%, and, optionally, from about 15% to about 25%, by weight of the liquid composition used to form the disintegrable film layer prior to drying.

After drying, the water insoluble polymer is present at a concentration of from about 35% to about 90%, optionally, from about 45% to about 80%, and, optionally, from about 60% to about 70%, by weight of the dry disintegrable film layer.

### The Disintegration Facilitator

### Plasticizers or Plasticizing Agents

The disintegrable film compositions of the present invention also comprise at least one disintegration facilitator selected from the group consisting of plasticizers or plasticizing agents, water insoluble particles or mixtures thereof.

Examples of suitable plasticizers include, but are not limited to, citric acid alkyl esters, glycerol esters such as glycerol monooleate and glycerol monostearate, phthalic acid alkyl esters, sebacic acid alkyl esters, sucrose esters, sorbitan esters, acetylated monoglycerides, glycerols, fatty acid esters, glycols, propylene glycol, and polyethylene glycols 200 to 12,000 and mixtures thereof. Specific plasticizers include, but are not limited to, lauric acid, triethyl citrate, acetyl triethyl citrate, triacetin (glyceryl triacetate), poloxamers, alkyl aryl phosphates, diethyl phthalate, tributyl citrate, dibutyl phthalate, dibutyl sebacate, polysorbate, Carbwax® series of polyethylene glycols (Union Carbide Corporation) and mixtures thereof.

In certain embodiments, the plasticizers can include mixtures of mono- and di-oleates supplied under name Atmos 300 by American Ingredients, Kansas City, MO.; triglycerides of caprylic/capric acids (or caprylic capric triglyceride) such as those sold by under the tradename Neobee 1053 by Stepan, Chicago, IL and mono- and di-glycerides of edible fats or oils supplied by Lonza Inc., Fair Lawn, NJ or Eastman Triacetin (food grade) supplied by Eastman Chemical Company, Kingsport, TN. The Atmos 300 and Neobee 1053 also function as releasing agents which aid in removal of the disintegrable layer from the casting sheet (e.g., polypropylene sheets) on which the layer is cast for drying.

When incorporated in the disintegrable film layer, the plasticizer is present at a concentration of from about 0.01% to about 10%, preferably from about 0.05% to about 8%, and most preferably from about 0.1 % to about 5% by weight of the liquid composition used to form the disintegrable film layer prior to drying.

In the above situation, after drying, the plasticizer is present at a concentration of from about 0.1% to about 25%, preferably from about 0.5% to about 15%, and most preferably from about 0.7% to about 10% by weight of the dry disintegrable film layer.

### Water Insoluble Particles

The disintegration facilitator can also be a water insoluble particle. Various kinds of organic powders and inorganic powders can be used as the water-insoluble particles.

The inorganic powders which are useful herein include, but are not limited to, microfine particles or granules of alumina, talc, magnesium stearate, titanium dioxide, barium titanate, magnesium titanate, calcium titanate, strontium titanate, zinc oxide, silica sand, clay, mica, tabular spar, diatomaceous earth, various inorganic oxide pigments, chromium oxide, cerium oxide, antimony trioxide, magnesium oxide, zirconium oxide, barium sulfate, barium carbonate, calcium carbonate, silica (colloidal or fumed), silicon carbide, silicon nitride, boron carbide, titanium carbide, and mixtures thereof.

The organic powders which are useful herein include cross- linked and non-cross-linked polymer powders, organic pigments, charge controlling agents, and waxes, for example. The cross-linked and non- cross-linked resin powders include, but are not limited to, resin powders of the styrene type, acrylic type, methacrylic type, polyethylene type, polypropylene type, silicone type, polyester type, polyurethane type, polyamide type, epoxy type, polyvinyl butyral type, for example. Mixtures of any of the above organic or inorganic powders can also be used. Additional particles useful in the present invention can be found in US patents 6,475,500; US 5,611,885; and US 4,847,199 each of which are herein incorporated by reference in its entirety.

US 6,475,500 discloses a non-spherical crosslinked siloxane elastomer having a particle size of from above 10 to about 200 microns having a viscosity of from above 20,000 to about 6,000,000 cps and is capable of absorbing greater than 30% by weight of a solvent fluid.

US 5,611,885 discloses particles which are added to fibers to give the fibers desired properties, such as, by way of example only, increased absorbency, abrasiveness, or antimicrobial activity. The particle can be any particulate material that has the desired property and which is capable of forming hydrogen bonds or coordinate covalent bonds with the binder.

In one disclosed embodiment in US 5,611,885, the added particles are superabsorbent particles, which comprise polymers that swell on exposure to water and form a hydrated gel (hydrogel) by absorbing large amounts of water. Superabsorbents are defined herein as materials that exhibit the ability to absorb large quantities of liquid, i.e. in excess of 10 to 15 parts of liquid per part thereof. These superabsorbent materials generally fall into three classes, namely starch graft copolymers, crosslinked carboxymethylcellulose derivatives and modified hydrophilic polyacrylates. Examples of such absorbent polymers are hydrolyzed starch-acrylonitrile graft copolymer, a neutralized starch-acrylic acid graft copolymer, a saponified acrylic acid ester-vinyl acetate copolymer, a hydrolyzed acrylonitrile copolymer or acrylamide copolymer, a modified cross-linked polyvinyl alcohol, a neutralized self-crosslinking polyacrylic acid, a crosslinked polyacrylate salt, carboxylated cellulose, and a neutralized crosslinked isobutylene-maleic anhydride copolymer.

Superabsorbent particles are available commercially, for example starch graft polyacrylate hydrogel fines (IM 1000F) from Hoechst-Celanese of Portsmouth, Va., or larger particles such as granules. Other superabsorbent particles are marketed under the trademarks SANWET (supplied by Sanyo Kasei Kogyo Kabushiki Kaisha), SUMIKA GEL (supplied by Sumitomo Kagaku Kabushiki Kaisha and which is emulsion polymerized and spherical as opposed to solution polymerized ground particles), FAVOR (supplied by Stockhausen of Greensboro, N.C.) and NORSOCRYL (supplied by Atochem). The superabsorbent particles come in a variety of sizes and morphologies, for example IM 1000 and IM 1000F. The 1000F is finer and will pass through a 200 mesh screen whereas IM 1000 has some particles that will not pass through a 60 mesh screen. Another type of superabsorbent particle is IM 5600 (agglomerated fines). Superabsorbent particulate hydrophilic polymers also are described in detail in US Pat. No. 4,102,340. That patent discloses hydrocolloid absorbent materials such as crosslinked polyacrylamides.

US 5,611,885 further discloses a number of water insoluble particulates in Table I.

US 4,847,199 discloses a number of water insoluble natural and synthetic particles including ferritin crystals, agarose particles, glass beads and polymeric particles such as latex particles. In particular, core-shell polymeric latex particles are disclosed having a core composed of homo-or copolymers of styrene, and a shell composed of homo- or copolymers of chloromethylstyrene.

The water insoluble particles of the present invention generally have a particle size or aggregate particle size of less than 10 microns, optionally, from about 0.01 microns to about 5 microns, optionally, from about 0.1 microns to about 1 micron, and, optionally, from about 0.1 to about 0.5 microns.

In certain embodiments, the insoluble particles can include Cabosil M-5 (fumed untreated silica) supplied by Cabot, Tuscola, IL.

When incorporated in the disintegrable film layer, the water insoluble particle is present at a concentration of from about 0.1 % to about 20%, optionally, from about 0.5% to about 10%, and, optionally, from about 1% to about 7% by weight of the liquid composition used to form the disintegrable film layer prior to drying.

In the above situation, after drying, the water insoluble particle is present at a concentration of from about 0.1 % to about 35%, optionally, from about 5% to about 20%, and, optionally, from about 10% to about 15% by weight of the dry disintegrable film layer.

The thickness of the first or disintegrable film layer can optionally range from about 1 micron to about 20 microns, optionally from about 3 microns to about 15 microns, optionally from about 5 microns to about 12 microns. The thicknesses of any additional layers can equal the range of thickness of the first or disintegrable layer or range from about 30 microns to about 150 microns, optionally from about 45 microns to about 130 microns, optionally from about 70 microns to about 120 microns.

Prior to drying, the compositions used to form the disintegrable layer include a solvent for the water insoluble polymer and the disintegration facilitator. Solvents useful in present invention are readily known and available to those skilled in the art. Suitable solvents include, but are not limited to, water; lower chained alcohols such as methanol, ethanol, n-propanol, isopropanol, n-butyl alcohol, sec-butyl alcohol, tert-butyl alcohol, ethyl acetate, methyl ethyl ketone, ethyl ether, hexane and mixtures thereof. The solvents are preferably present in compositions used to form the film layer of the present invention at sufficient amounts, before drying, to dissolve the components with which it is mixed.

### The Whitening Composition Layer.

The whitening composition layer is be disposed or laminated onto the surface of the disintegrable film composition. In certain embodiments, the whitening composition layer is fixed to a pre-formed disintegrable layer by casting a layer of liquid whitening composition onto the disintegrable layer and then drying the bi-layer product.

In certain embodiments, the whitening composition layer or at least one of the whitening composition layers (in a multi-layer strip) is generally formulated to be a dry, non-sticky carrier layer for teeth whitening actives.

Additionally, because of the brittleness of water insoluble polymers in certain embodiments, the disintegrable layer of the present invention typically lacks structural integrity. Under these circumstances, the disintegrable layer must be supported by a support layer (such as a polypropylene or polyethylene liner) or on the whitening composition layer of the present invention before it can be handled or manipulated without disintegrable layer flaking or breaking apart.

The total weight of the disintegrable layer is from about 1mg to about 25mg, optionally, from about 5mg to about 15mg, and optionally about 12 mg. The disintegrable layer avoids contact between the whitener composition layer and the fingers during handling. Forming the disintegrable layer from water insoluble polymers prolongs the residence time of the disintegrable layer. Specifically, during use, it slows, but does not prevent, hydration of the adhesive layer and it does not prevent saliva or surrounding soft tissue from coming into contact with the whitening composition layer. The use of water insoluble polymers in the disintegrable layer also avoids the gummy feel on the lips, tongue, and other soft tissue in the mouth typically associated with film layers formed using water soluble polymers.

Upon hydration of the whitening composition layer in the mouth, the water permeability of the disintegrable layer is improved.

Typically, the disintegrable layer is supported by the whitening composition layer, as the disintegrable layer has insufficient structural integrity to be handled unless it is adhered to the whitening composition layer or to some other support. Following application to teeth, the disintegrable layer cannot be reformed to its original shape and size because it fractures and disintegrates. The function of the components of the disintegrable layer in addition to the shellac is to facilitate passage of saliva through the disintegrable layer and into the whitening composition layer.

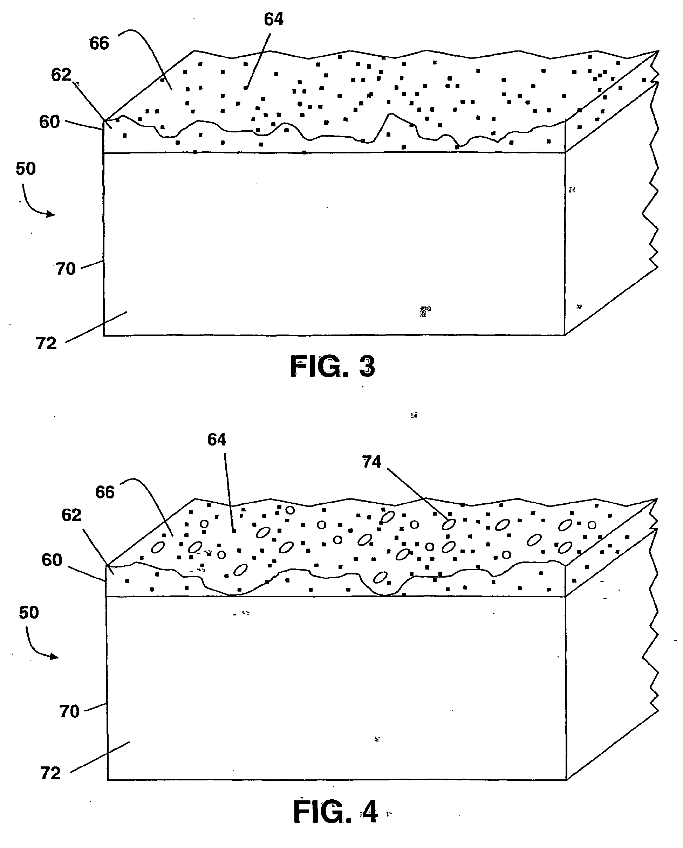

When attached to the whitening composition layer, the water permeability of the disintegrable layer is improved once the whitening composition layer is hydrated. Without being limited by theory, it is believed that the improved permeability of the disintegrable layer may be attributed to one or more of the following factors:
■ The wicking action of such water insoluble particles as fumed silica;
■ Capillary action caused by the hydrated whitening composition layer; and
■ The presence of substantial discontinuous empty domains (i.e., no water insoluble polymer - See Figure 4) along the surface of the cast disintegrable layer.
Also, without being limited by theory, it is further believed that because of the general brittleness of many of the water insoluble polymers, the thinness of the disintegrable layer, and the presence of the water insoluble particles combined with the rolling /unrolling of the disintegrable layer during the casting process, there is the potential for cracking in the disintegrable layer. This cracking may also contribute, to some extent, to the improved water permeability of disintegrable layer.

While still not being limited by theory, it also believed that the above factors together with the mechanical action of the lips, tongue and other soft tissue also result in the improved disintegration properties of the disintegrable layer.

The whitening composition layer comprises one or more teeth whitening actives Suitable teeth whitening active are selected from the group consisting of oxalates, peroxides, metal chlorites, perborates, percarbonates, peroxyacids, and mixtures thereof. Suitable peroxide compounds include: hydrogen peroxide, calcium peroxide, sodium peroxide, carbamide peroxide, urea peroxide, sodium percarbonate and mixtures thereof. Optionally, the peroxide is hydrogen peroxide. Suitable metal chlorites include calcium chlorite, barium chlorite, magnesium chlorite, lithium chlorite, sodium chlorite and potassium chlorite. Additional whitening actives may be hypochlorite and chlorine dioxide. A preferred chlorite is sodium chlorite. The effectiveness of whitening actives can, optionally, be enhanced by means of a catalyst, i.e. a two-component peroxide- catalyst; system. Useful whitening agent catalysts or catalytic agents can be found in US 6,440,396 to McLaughlin, Gerald, herein incorporated by reference in its entirety. A "catalytic agent" is a compound or molecule which accelerates the whitening action of the bleaching compound without being consumed in the reaction. In a preferred embodiment, the catalytic agent accelerates the release of oxygen radicals form an oxygen radical generating agent. Examples of such agents include, but are not limited to activated charcoal, platinum, platinum salts, copper, copper salts, palladium, palladium salts, silver, and silver salts. In a preferred embodiment, activated charcoal is used as the catalytic activator. Of particular use is the commercially activated charcoal Centaur, produced by Calgon, Inc.

The whitening composition layer can optionally take the form of a peroxide-containing gel. Suitable gels may be based on glycerol containing a peroxide such as hydrogen peroxide or an organic peroxide. A suitable gel is that disclosed in US-A-3,657,413, for example that sold under the trade mark PROXIGEL by The Block Drug Company (USA) (since acquired by GlaxoSmithKline plc).

US 3,657,413 discloses antiseptic compositions that comprise urea peroxide, glycerol and a carboxypolymethylene polymer or salts thereof, the latter being incorporated in the formulation in an amount sufficient to provide a gel having a viscosity of at least about 1000 centipoises at room temperature.

Other suitable peroxide-containing gels are for example disclosed in the art references cited above. The film may have the topical or system active deposited upon its surface.

The teeth whitening active is typically present at a concentration of from about 0.1 % to about 30%, optionally, from about 1% to about 25%, and, optionally, from about 5% to about 15% by weight of the teeth whitening composition layer after drying.

When incorporating peroxide actives, the whitening composition layer of the present invention can, optionally, contain peroxide active stabilizers. Peroxide active stabilizers suitable for use herein include, but are not limited to polyethylene glycols such as PEG 40 or PEG 600; zinc salts such as zinc citrate; polyoxyalkylene block-polymers (e.g., Pluronics); aminocarboxylic acids or salts thereof; glycerols; dyes such as Blue #1 or Green #3; phosphates such as phosphoric acid, sodium phosphate or sodium acid pyrophosphate; stannous salts such as stannous chloride; sodium stannate; citric acid; etidronic acid; carbomers or carboxypolymethylenes such as those of the Carbopol® seriers, butylated hydroxytoluene (BHT), ethylenediaminetetraacetic acid (EDTA) and mixtures thereof.

The whitening composition layer additionally comprises one or more water soluble polymers. In certain embodiments, the water soluble polymer is non-sticky when dry, yet sticky or tacky when hydrated - for example by saliva on the teeth. Example of suitable water soluble polymers include, but is not limited to, polyvinyl alcohol, polyvinyl pyrrolidone (PVP), carrageenan, locust bean gum, guar gum, hydroxy ethyl cellulose, xanthan gum, tragacanth gum, pullulan, starch and mixtures thereof. Also useful herein are the hydrophilic glass polymers described in US patent 6780401 to Kim et al., herein incorporated by reference in its entirety.

US 6,780,401 discloses hydrophilic glass polymers that may be selected to have a good compatibility with peroxide. Suitable polymers include poly alkyl vinyl ether-maleic acid copolymer (PVM/MA copolymer) such as, Gantrez AN 119, AN 139, and S-97, polyvinyl alcohol, polyacrylic acid, Poloxamer 407 (Pluronic), polyvinyl pyrrolidone-vinyl acetate copolymer (PVP/VA copolymer), such as Luviskol VA, and Plasdone S PVP/VA, polyvinyl pyrrolidone (PVP, K-15~K-120), Polyquaterium 11 (Gafquat 755N), Polyquaterium 39 (Merquat plus 3330), carbomer (Carbopol), hydroxyl propyl methyl cellulose, hydroxyl ethyl cellulose, hydroxyl propyl cellulose, gelatin and alginate salt such as sodium alginate. The above-described polymers can be used alone or in a mixture. A solvent for these polymers includes water, ethanol or any mixture thereof.

In certain embodiments, the water soluble polymer is a PVP having a weight average molecular weight of from about 3,000 to about 2,000,000, optionally from about 10,000 to about 1,600,000, and optionally from about 100,000 to about 1,500,000 daltons as measured using light scattering method.

Suitable PVP's are supplied by ISP Company under the tradename Plasdone. In certain embodiments, PVP K-90 (weight average molecular weight of 1.3 million daltons as measured using light scattering method) is used.

When incorporated in the whitening composition layer of the present invention, the water soluble polymer is present at a concentration of from about 0.1% to about 30%, optionally, from about 1% to about 25%, and, optionally, from about 5% to about 20% by weight of the liquid composition used to form the whitening composition layer prior to drying.

In the above situation, after drying, the water soluble polymer is present at a concentration of from about 20% to about 80%, optionally, from about 30% to about 60%, and, optionally, from about 40% to about 50% by weight of the dry whitening composition layer.

Optionally, plasticizers may be incorporated into the whitening composition layer of the teeth whitening device. Suitable plasticizers include those previously listed above. The plasticizers may be incorporated at levels of from about 0.01% to about 3%, and optionally from about 0.1 % to about 1% by weight of the wet film composition.

In certain embodiments, the whitening composition layer comprises additional oral care actives. The level of oral care active in the present invention may generally be from about 0.01% to about 40% or, optionally, from about 0.1% to 20% by weight of the whitening composition layer after drying.

Essential oils may be included in or associated with the whitening composition layer the present invention. Essential oils suitable for use herein are described in detail in US patents 6,596,298 to Leung et al., previously incorporated by reference in its entirety.

These essential oils include thymol, methyl salicylate, menthol and eucalyptol. Generally the amount of thymol, methyl salicylate and eucalyptol is from about 0.01 to about 4 wt% of the film composition, preferably about 0.50 to about 3.0 wt% and even more preferably from about 0.70 to about 2.0 wt% of the film. Menthol can be added from about 0.01 to about 15 wt% of the composition, preferably about 2.0 to about 10 wt% and even more preferably from about 3 to about 9 wt% of the film. The amounts added can be readily determined to those skilled in the art and can exceed these amounts as long as the total oil content does not create sticking or other processing problems. In certain embodiments, the essential oils are combined in amounts synergistically effective to kill the plaque-producing germs that cause dental plaque, gingivitis and bad breath.

An additional carrier material may also be added to the film composition of the present invention. These materials can be added as additional components for properties other than those previously mentioned and can include humectants and include glycerin, sorbitol, polyethylene glycol and the like. The film composition may comprise the substance itself, together with one or more substance enhancers, for example catalysts and/or potentiators to modify the release and/or activity of the substance.

The whitening composition layer of the invention may additionally comprise additional substances such as flavors, colors, etc. which may for example be deposited onto the surface of the whitening composition layer (or either surface of the teeth whitening device) or impregnated into the bulk of the whitening composition layer (or the teeth whitening device).

A pH adjusting agent may also be added to optimize the storage stability of the gel and to make the substance safe for the oral tissues. These pH adjusting agents, or buffers, can be any material which is suitable to adjust the pH of the oral care substance. Suitable materials include sodium bicarbonate, sodium phosphate, sodium hydroxide, ammonium hydroxide, sodium stannate, triethanolamine, citric acid, hydrochloric acid, sodium citrate, and combinations thereof. The pH adjusting agents are added in sufficient amounts so as to adjust the pH of the whitening composition layer prior to drying to a suitable value, e.g. about 4.5 to about 11, preferably from about 5.5 to about 8.5, and more preferably from about 6 to about 7. The pH adjusting agents are generally present in an amount of from about 0.01% to about 15% and preferably from about 0.05% to about 5%, by weight of the composition used to form the whitening composition layer prior to drying.

For example a teeth whitening gel may be deposited directly as a layer on the surface of a disintegrable layer as described above. Alternatively a gel may be absorbed into the disintegrable layer, or impregnated into the bulk of the film material, or deposited between layers of a multiple layered film.

Methods of depositing substances upon the surfaces of layers separately or the tooth whitening device generally as described above are known, for example printing, e.g. silo screen printing, passing between impregnated rollers, dosing, a pump and nozzle, spraying, dipping etc. Methods of impregnating substances into the bulk of film materials are also known, for example admixing the substance into the strip material and then forming the strip, or exposure of the strip to the substance under conditions which cause the substance to be impregnated into the strip. Alternatively, one example of the film material may be a foam material, particularly an open-cell foam material, and the substance may be impregnated into the strip material by introducing the substance into the cells of the foam.

In another embodiment, the disintegrable film layer of the present invention forms the first layer of a bilayer on to which is dried a second layer where the second layer is a whitening composition layer comprising water soluble polymers such as that described in US patents 6,596,298 to Leung et al. and 6,419,903 to Xu et al., both of which are herein incorporated by reference in their entirety. The bilayer film is then applied to the teeth and allowed to erode over time in the presence of saliva or other aqueous media.

Suitable water soluble polymer film layers are disclosed in US 6,596,298 to Leung et al. US 6,596,298 discloses a consumable film that adheres to and dissolves in a mouth of a consumer, wherein said film is a single layer, water-soluble solid comprising pullulan and an antimicrobial effective combination of at least two essential oils selected from the group consisting of (i) about 0.01 to about 4 wt% thymol, (ii) about 0.01 to about 4 wt% methyl salicylate, (iii) about 0.01 to about 4 wt% eucalyptol and (iv) about 0.01 to about 15 wt% menthol, wherein at least thymol and menthol are present in said film.

The film preferably comprises pullulan, thymol, methyl salicylate, eucalyptol and menthol.

Generally the amount of thymol, methyl salicylate and eucalyptol is from about 0.01 to about 4 wt% of the film composition, preferably about 0.50 to about 3.0 wt% and even more preferably from about 0.70 to about 2.0 wt% of the film. Menthol can be added from about 0.01 to about 15 wt% of the composition, preferably about 2.0 to about 10 wt% and even more preferably from about 3 to about 9 wt% of the film. The amounts added can be readily determined to those skilled in the art and can exceed these amounts as long as the total oil content does not create sticking or other processing problems. In certain embodiments, the essential oils are combined in amounts synergistically effective to kill the plaque-producing germs that cause dental plaque, gingivitis and bad breath.

The film-forming agent used in the films can be selected fro the group consisting of pullulan, hydroxypropylmethyl cellulose, hydroxyethyl cellulose, hydroxypropyl cellulose, polyvinyl pyrrolidone, carboxymethyl cellulose, polyvinyl alcohol, sodium alginate, polyethylene glycol, xanthan gum, tragacanth gum, guar gum, acacia gum, arabic gum, polyacrylic acid, methylmethacrylate copolymer, carboxyvinyl polymer, amylose, high amylose starch, hydroxypropylated high amylose starch, dextrin, pectin, chitin, chitosan, levan, elsinan, collagen, gelatin, zein, gluten, soy protein isolate, whey protein isolate, casein and mixtures thereof. A preferred film former is pullulan, in amounts ranging from about 0.01 to about 99 wt%, preferably about 30 to about 80 wt%, more preferably from about 45 to about 70 wt% of the film and even more preferably from about 60 to about 65 wt% of the film.

The film preferably comprises pullulan as a film-forming agent and the essential oils as antimicrobial/flavoring agents, and can further comprise water, additional antimicrobial agents, additional film-forming agents, plasticizing agents, additional flavoring agents, sulfur precipitating agents, saliva stimulating agents, cooling agents, surfactants, stabilizing agents, emulsifying agents, thickening agents, binding agents, coloring agents, sweeteners, fragrances, and the like.

Due to the relatively high oil content in the oral care film, it is preferable to avoid substantial amounts of humectant in the film (and more preferable to have no humectant in the film), so as to avoid producing an overly moist, self-adhering film. In particular, it is preferred to formulate the film with a plasticizing agent other than glycerin, which is also a humectant, and with a sweetener other than sorbitol, which is a mild humectant.

A preferred consumable film comprises about 40 to about 80 wt% pullulan; about 0.01 to about 4 wt% thymol; about 0.01 to about 4 wt% methyl salicylate; about 0.01 to about 4 wt% eucalyptol; and about 0.01 to about 15 wt% menthol.

US 6,419,903 discloses an orally consumable film composition for delivering breath freshening agents to the oral cavity which is rapidly dissolvable in the oral cavity, the composition being comprised of a homogeneous mixture of (1) a water soluble, low viscosity hydroxyalkylmethyl cellulose, the viscosity being in the range of 1 to about 50 mPa·s as determined as a 2% by weight aqueous solution at 20°C using a Ubbelohde tube viscometer, (2) a water dispersible pregelatized starch, and (3) a flavoring agent.

The film of US 6,419,903 comprises hydroxyalkylmethylcellulose as a film forming agent combined with starch, and a flavoring agent particularly an essential oil as the breath freshening agent. The film can further comprise water, additional film forming agents, plasticizing agents, flavoring agents, antimalodor agents, surfactants, emulsifying agents, coloring agents, sweeteners and fragrances.

In preparing the film, a low viscosity hydroxyalkylmethyl cellulose, a starch ingredient, a breath freshening agent and other film forming ingredients are dissolved in a compatible solvent to form a film forming composition. The hydroxyalkyl cellulose to starch ratio (by weight) may vary from about 1:3 to about 4:1 and preferably about 1:1.5 to about 2.5:1.

The composition is cast on a releasable carrier and dried. The carrier material must have a surface tension which allows the film solution to spread evenly across the intended carrier width without soaking to form a destructive bond between the film and carrier substrates. Examples of suitable carrier materials include glass, stainless steel, Teflon and polyethylene-impregnated paper. Drying of the film may be carried out at high temperature using a drying oven drying terminal, vacuum drier, or any other suitable drying equipment which does not adversely effect the ingredients of which the film is composed.

The film forming agent used in the films according to US 6,419,903 is preferably a low viscosity hydropropylmethyl cellulose polymer (HPMC). It is critical that the HPMC have a viscosity in the range of about 1 to about 40 millipascal seconds (mPa·s) as determined as a 2% by weight aqueous solution of the HPMC at 20°C using a Ubbelohde tube viscometer. Preferably the HPMC has a viscosity of about 3 to about 20 mPas at 20°C.

Cold water swellable, physically modified and pregelatinized starches are particularly useful as texture modifier to increase the stiffness of the hydroxyalkyl methyl cellulose polymer films, as the breath film prepared by HPMC alone, at the thicknesses described, tends to curl up after it is cast and dried. To prepare such starch products, the granular starch is cooked in the presence of water and possibly an organic solvent at a temperature not higher than 10°C higher than the gelatinization temperature. The obtained starch is then dried.

Additionally the film layers of the present invention can be manufactured using hot melt extrusion techniques such as that described in US patent 6,375,963 B1 to Repka et al. herein incorporated by reference in its entirety.

US 6,375,963 provides a process for preparing a hot-melt extruded film comprising the steps of:
providing a precursor composition comprising about 50-99% wt. of one or more water soluble or water swellable polymers, about 0-50% wt., or up to 50% wt. of a therapeutic agent; and about 1-25% wt., or up to 25% wt., of a bioadhesive polymer; and
hot-melt extruding the precursor composition to form a film;
wherein the precursor composition is hot-melt extruded without extensive degradation in the absence of a plasticizer.

The device of the invention may be marked with one or more visible symbol, e.g. text matter, a trade mark, a company logo, an area of color, or an alignment feature such as a visible line or notch etc. to assist the user in applying the device to the teeth in a proper alignment. Such an alignment feature may for example comprise a symbol to show the user which way up the device should be whilst applying the device to the teeth, or which of a pair of the devices is intended for the upper teeth and which for the lower teeth. This way the device may be made more visually attractive and/or easier to use. Such symbol(s) may be applied by conventional printing or embossing processes, e.g. silk screen printing, inkjet printing etc. to the surface of the plastically deformable material opposite to the surface on which is attached the layer of an absorbent material.

If such a visible symbol is applied to this surface, a cover layer can, optionally, be applied over the symbol, for example to protect it. This cover layer may be transparent or translucent to allow visible symbols to be seen through this layer. Such a cover layer can, optionally, be applied to the film by pressing, e.g. rolling, the material of the cover layer in contact with the film.

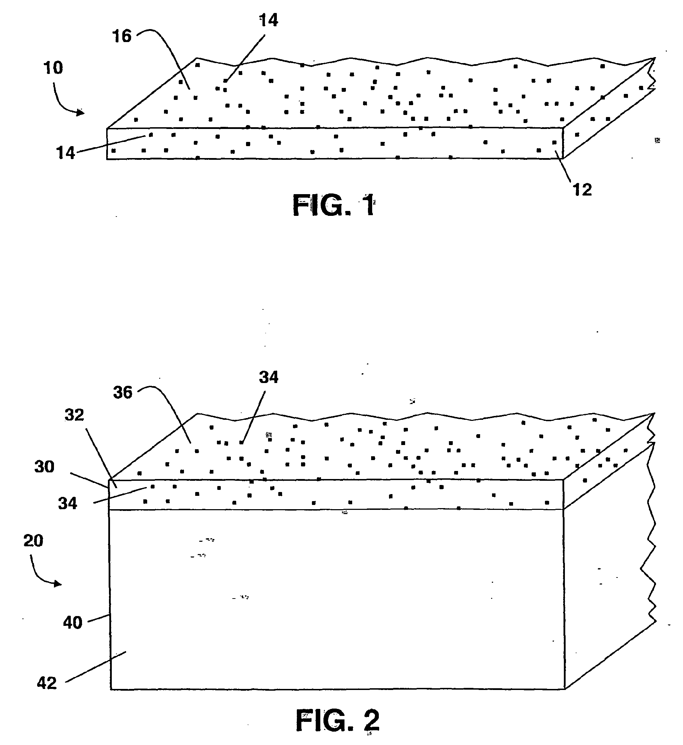

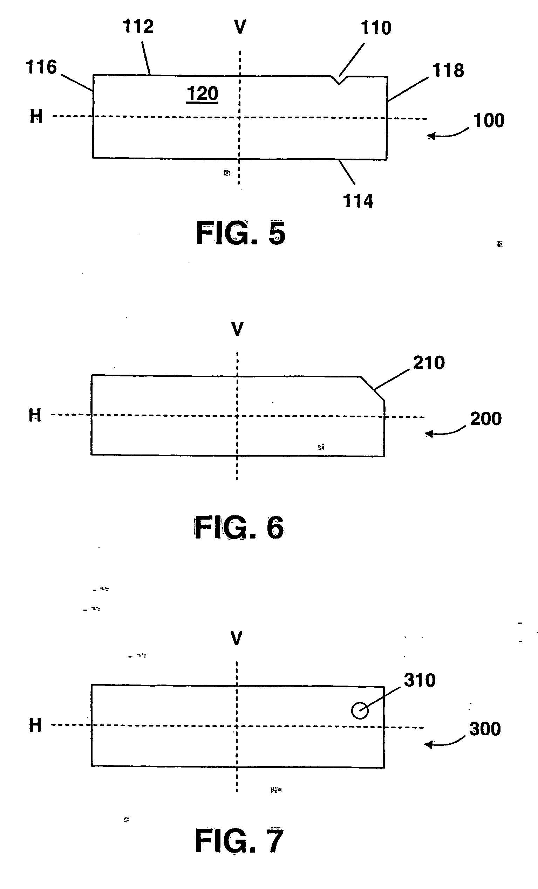

Turning to the drawings, exemplary films **10, 20,** and **50,** are illustrated in FIGURES 1-4, and exemplary film products **100, 200, 300, 400, 500,** and **600** are illustrated in FIGURES 5-10.

FIGURE 1 shows a single layer film embodiment of the present invention. The figure shows film **10** comprised of water insoluble polymer **12** and disintegration facilitator **14.** Disintegration facilitator **14** may be fully incorporated into water insoluble polymer **12,** may be only at the surface **16** of film **10,** or, as shown in the present embodiment, may be both fully incorporated into water insoluble polymer **12,** as well as present at surface **16** of film **10.** FIGURE 1 shows disintegration facilitator **14** uniformly distributed in water insoluble polymer **12.** However, it is to be understood that the distribution of disintegration facilitator **14** in insoluble polymer **12** may be non-uniform. More or less disintegration facilitator **14** may be present at surface **16** of film **10** or in the bulk of insoluble polymer **12.**

FIGURES 2-4 show bi-layer film embodiments **20** and **50** of the present invention. FIGURE 2 shows film **20** comprised of first layer **30** and second layer (i.e., an active composition layer) **40.** First layer 30 is comprised of water insoluble polymer **32** and disintegration facilitator **34.** Disintegration facilitator **34** may be fully incorporated into water insoluble polymer **32,** may be only at the surface of first layer **30,** or, as shown in the present embodiment, may be both fully incorporated into water insoluble polymer **32,** as well as present at surface **36** of first layer **30.** Second layer **40** can be comprised of polymer. In one another embodiment, second layer **40** is an oral care active in a water soluble polymer **42** film layer such as that described in US patents 6,596,298 to Leung et al. and 6,419,903 to Xu et al., both of which are herein incorporated by reference in their entirety. The bi-layer film is then applied to the teeth and allowed to erode over time in the presence of saliva or other aqueous media and the mechanical action of the lips, tongue and other soft tissue.

FIGURES 3 and 4 show alternative bi-layer film embodiment **50** of the present invention. FIGURE 3 shows film **50** comprised of first layer **60** and second layer **70.** First layer **60** is comprised of water insoluble polymer **62** and disintegration facilitator **64.** Disintegration facilitator **64** is shown to be both fully incorporated into water insoluble polymer **62,** as well as present at surface **66** of first layer **60.** Second layer **70** is comprised of peroxide active and water soluble polymer **72.** In the embodiments shown in FIGURES 3 and 4, the thickness of first layer **60** is not uniform, resulting in an uneven surface **66** of first layer **60.** FIGURE 3 shows an embodiment in which first layer **60** fully covers second layer **70,** leaving no exposed sections. FIGURE 4 shows an embodiment in which first layer **60** does not fully cover second layer **70** leaving exposed or virtually exposed sections **74.**

Though only single layer and bi-layer film embodiments of the present invention are shown, it should be understood that film embodiments of the present invention may also be in the form of multi-layer films, where one or more films such a described herein are laminated with other films, foams, or gels to build film products.

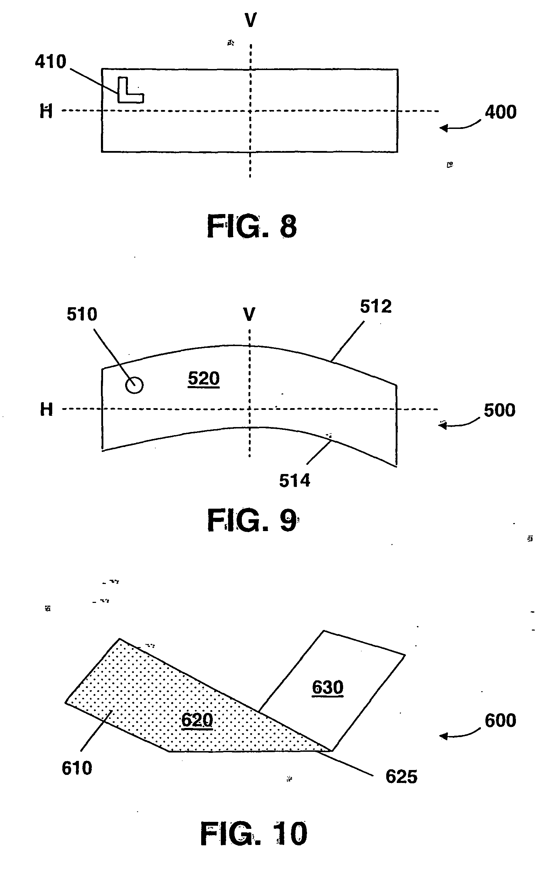

Single layer and multi-layer films as discussed above will be used in a variety of film products. Exemplary film products **100, 200, 300, 400, 500,** and **600** are illustrated in FIGURES 5-10, with exemplary respective positioning features **110, 210, 310, 410, 510,** and **610.** In the following description, elements or components similar to those in the embodiment of Figs. 5 to 10 (though not necessarily having identical features) are designated with the same reference numbers increased by 100 for each increase in figure number and redundant description is omitted. It will be appreciated that the film product may be in any desired shape or form, and may be made of any desired material or composition. Thus, although the exemplary film products **100, 200, 300,** and **400** illustrated in FIGURES 5-8 are rectangular, it will be appreciated that the principles of the present invention may be applied to a film product of any other desired shape, such as round, square, triangular, trapezoidal, irregular, etc. For instance, a film product may be formed with curvilinear sides, such as film product **500** of FIGURE 9.

It will further be appreciated that the positioning feature to be provided on a film product in accordance with the principles of the present invention may be in any desired shape or form that achieves the desired purpose of distinguishing or differentiating features or aspects of the film product for proper use and/or application and/or functioning of the film product. For instance, the positioning feature preferably may be provided to identify a particular orientation of the film product itself, such as by identifying which surface is facing the user and which surface is facing the treatment site. The positioning feature may alternatively or additionally be provided simply for purposes of alignment at and/or with respect to the treatment site. The manner in which the positioning feature is provided, such as the form or location, need not be limited to the exemplary embodiments of FIGURES 5-10. Instead, it will be appreciated that the basic function of the positioning feature as distinguishing or differentiating features or aspects of the film product may be achieved without restricting the precise form of the positioning feature.

Turning to the exemplary embodiments of FIGURES 5-10, a positioning feature formed in accordance with the principles of the present invention may be formed in any of a variety of manners, including, without limitation, a mechanical / structural identifier, a visual indicator on a surface, or a textural feature on a surface. The film product may be provided with a positioning feature by being shaped or marked with a shape, cut-out, figure, color, hologram, mark, word, texture, or other indicia, which can uniquely identify sufficient information about the film product for the desired manipulation and/or orienting and/or general use of the film product. The type of positioning feature, as well as its location, may dictate the feature-distinguishing and indicating functions the positioning feature serves.

One simple embodiment of a positioning feature in accordance with the principles of the present invention is a mechanical or structural identifier (hereinafter, simply "mechanical identifier" for the sake of convenience and without intent to limit such term) such as positioning features **110, 210, 310, 410, 510** of FIGURES 5-9. Positioning features **110, 210, 310, 410, 510** are formed by mechanically altering the structure of the film product **100, 200, 300, 400, 500.** For instance, an irregularity may be formed along an edge of the film product, as in FIGURES 5 and 6, or a cut-out may be formed through the film product spaced from the edges of the film product, as in FIGURES 7-9. More particularly, an irregularity along an edge of the film product may be formed in any desired shape, such as a notch **110** (FIGURE 5) or a cut-off corner **210** (FIGURE 6). Similarly, a cut-out may be formed in any desired shape, such as a round hole **310, 510** (FIGURES 7 and 9) or hole of another shape, such as an asymmetrical shape like L-shaped hole **410** (FIGURE 8).

Positioning features in the form of a mechanical identifier by their very nature can indicate orientation of the film product with respect to a treatment site. However, a simple mechanical identifier cannot necessarily definitively indicate the orientation of the surfaces of the film product itself. This is because the presence alone of such an indicator as a mechanical identifier may not provide sufficient data to a user to be able to differentiate at least certain features of the film product. For example, if a film product is symmetrical about an axis of symmetry, then a mechanical identifier positioned along such axis of symmetry (especially a mechanical identifier that itself is symmetrical about such axis of symmetry as well) cannot necessarily serve to differentiate the opposing surfaces of the film product from each other. As another example, a round hole provided as a positioning feature in a round film product would not necessarily provide sufficient information to differentiate the opposing surfaces of the film product.

One manner of allowing a positioning feature in the form of a mechanical identifier to differentiate, more definitively, the surfaces of a film product is to locate the mechanical identifier at a readily identifiable position on the film product itself. Generally, the ability of a mechanical identifier to distinguish a surface of a film product is facilitated by basing selection of the location of the mechanical identifier on the shape of the film product. One useful principle is to place the mechanical identifier offset from any and all axes of symmetry. As discussed above, if the mechanical identifier is along an axis of symmetry of a film product it is difficult, if not impossible, to differentiate the opposite surfaces of the film product. Another useful principle is that the shape of the mechanical identifier can serve as a further indicator of the orientation of the film product on which the mechanical identifier is provided. Such principles may be better appreciated with reference to the exemplary embodiments of FIGURES 5-8, as will now be described.

Exemplary film products **100, 200, 300,** and **400** have shapes that are symmetrical about a horizontal central axis and also about a vertical central axis. Accordingly, it is desirable to locate a positioning feature **110, 210, 310, 410** in the form of a mechanical identifier offset from both the horizontal and vertical central axes so that the mechanical identifier may serve to differentiate the symmetrical sides of the film product. Reference is made to film product **100** of FIGURE 5 to illustrate this principle. Film product **100** has two pairs of opposite edges: opposite edges **112** and **114** (respectively top and bottom edges according to the orientation of film product **100** in FIGURE 5), and opposite edges **116** and **118** (respectively left and right edges according to the orientation of film product **100** in FIGURE 5). Positioning feature **110** is formed along top edge **112** at a location offset from central vertical axis **V.** In other words, positioning feature **110** is not located at a midpoint along the length of top edge **112.** Moreover, because top edge **112** is, by its very nature as an edge, offset from central horizontal axis **H,** positioning feature **110,** when provided along top edge **112,** necessarily is also offset from central horizontal axis **H** as well. Accordingly, a user may be assured that when looking at film product **100** if positioning feature **110** is on the right side of top edge **112** of film product **100,** then the surface **120** seen in FIGURE 5 is the surface facing the user at that point. Rotation of film product **100** within the plane of the page does not alter such clear position indicating capability. For instance, if positioning feature **110** is located along what appears to be a bottom edge, on the left side thereof, it is also clear that surface **120** is facing the user. In contrast, if positioning feature **110** appears on the left side of top edge **112,** then it is clear to the user that the surface opposite surface **120** is facing the user.

In contrast, exemplary film product **500** of FIGURE 9 illustrates a film product that is symmetrical about only one axis of symmetry (vertical central axis **V).** The asymmetrical sides of film product **500** are inherently distinguishable from one another. Accordingly, positioning feature **510** need only be positioned offset from the axis of symmetry, axis **V.** So long as positioning feature **510** appears on the left side of film product 500 when convex edge **512** is a "top" edge and concave edge **514** is a "bottom" edge, it is clear that the surface **520** of film product 500 seen in FIGURE 9 is the surface facing the user at that point as well.

Of course, complete symmetry, such as in a circular film product, complicates placement, as described above with respect to provision of a round hole as the positioning feature for a round film product. An asymmetrical positioning feature, such as the L-shaped positioning feature **410** of film product **400** in FIGURE 8, may be used advantageously to permit orientation of the asymmetrical positioning feature to distinguish symmetrical sides from one another and thus to permit differentiation of the surfaces of a symmetrical film product.

As noted above, instead of providing a positioning feature in the form of a mechanical identifier, a positioning feature may be formed in accordance with the principles of the present invention as a surface feature which alters the surface of the film product. If the positioning feature is formed on a surface of the film product, then such positioning feature is helpful in differentiating opposing surfaces of the film product. In general, in contrast with a structural feature, a surface feature preferably does not extend through the material of the film product. Such a surface feature may be in any desired form that permits either visual or tactile differentiation of the surfaces of the film product. For instance, a visual indicator such as visible symbols, printed indicia, stippling, shading, or coloring may be used for ready visual differentiation of the surface bearing such visual indicator. Such visual indicator may be applied by any desired technique, including, without limitation, blotching, coloring, cutting, embossing, engraving, marking, printing (by ink jet, video jet, or flexographic printing, or any other desired technique known in the art), shaping, stamping. Additionally, or alternatively, surface texturing that alters the tactile features of the surface, such as by formation of non-smooth, unsmooth, or rough areas or regions ("textured" hereafter for the sake of convenience and without intent to limit), may be used to differentiate surfaces of the film product via tactile sensation.

Positioning features in the form of surface features are generally useful for film products that carry a particular active on only one surface thereof and only deliver the active at or via that surface. Such positioning feature may be particularly helpful in instances in which the surfaces are not readily distinguishable from each other. Examples of film products having surfaces that are not readily distinguishable include film products utilizing a dry adhesive such that a releasable release strip pr liner is not needed to protect a sticky adhesive surface and thus cannot provide an indication of which surface bears the adhesive and is to be applied to the treatment site.

An exemplary embodiment utilizing a surface feature as a positioning feature is illustrated in FIGURE 10. Film product **600** of FIGURE 10 is illustrated folded over itself to show opposite surfaces **620** and **630.** As may be appreciated, positioning feature **610** is a surface feature provided on only one of the surfaces of film product **600.** As such, surface 620, bearing positioning feature **610,** is readily distinguishable from surface **630.**

Thus, it will be appreciated that the present invention facilitates use and/or application of a film product upon the user's location or identification of a positioning feature provided on the film product in accordance with the principles of the present invention. Once the positioning feature has been located or identified, the user can now differentiate or distinguish at least one feature of the film product. Such information may be employed by the user to use and/or manipulate and/or orient and/or apply the film product as desired. Film products incorporating positioning features in accordance with the principles of the present invention may be sold with instructions for the end users regarding locating the positioning feature, and identifying a desired feature of the film product based on information obtainable upon locating the positioning feature. The instructions may further provided guidance as to use of the film product in conjunction with location of the positioning feature.

As discussed earlier, it will be appreciated that the film products **100, 200, 300, 400, 500** may be any type of film product preferably configured to provide a therapeutic affect at a desired treatment site. One example of a film product that may embody the principles of the present invention is a medicated strip for delivering a systemic or topical active to a treatment site. A more specific example of such a medicated strip is one applied to teeth to whiten the teeth. The positioning features of the present invention are particularly helpful in assisting the user in orienting the medicated strip for proper application to his/her teeth. One exemplary tooth whitening strip utilizes a dry, moistenable adhesive and therefore can be applied directly to the user's teeth upon removal from a protective packaging or pouch without the need to remove a releasable release strip/liner or otherwise to alter the structure or material of the tooth whitening strip. Provision of a positioning feature on the tooth whitening strip facilitates orientation of the strip, which is essential if only one of the surfaces can deliver the whitening agent. It will, however, be appreciated that the principles of the present invention may be applied to film or strip products for use other than in oral cavities.

### Process of Making

A whitening strip of the present invention is prepared by mixing the components of the disintegrable layer and separately mixing the component of the whitening composition layer. The disintegrable layer is cast onto a polypropylene (or similar) nonstick surface or sheet/liner whereby the disintegrable layer is dried. The clarity of the disintegrable layer is improved by passing the mixed components of the disintegrable layer through an homogenizer.

After casting and drying the disintegrable layer, the polypropylene sheet/liner with the disintegrable layer is then rolled-up for subsequent processing. A whitening composition layer is subsequently poured on top of the disintegrable layer, as the disintegrable layer is unrolled. After drying the whitening composition layer, the polypropylene sheet or liner is removed by mechanical peeling prior to packaging. Until the adhesive layer is dried and thereby attains structural integrity, the disintegrable layer must be supported by the polypropylene sheet or liner. Optionally, a notch or some other position orienting symbol is added to assist the user in positioning the strip so the whitening composition layer contacts the teeth during use.

### Methods for Delivering Whitening Actives

The present invention can be used where retention of whitening actives is required for whitening or bleaching activity. Generally, the delivery of the teeth whitening actives involves topically applying the inventive whitening product containing a safe and containing effective amount of such actives to a tooth or teeth in a manner described in US patents 5,894,017; 5,891,453; 6,045,811; and 6,419,906, each of which is herein incorporated by reference in its entirety. In certain embodiments, the tooth whitening device is sized to only fit the teeth and as such is applied at or below the gumline. Preferably the whitening compositions layer of the tooth whitening device are not directly applied to the gum. The frequency of application and the period of use will vary widely depending upon the level of treatment required or desired, e.g., the degree of teeth whitening desired.

### Examples

The teeth whitening product or device is illustrated in following examples illustrate specific embodiments of the teeth whitening device of the present invention, but are not intended to be limiting thereof. Other modifications can be undertaken by the skilled artisan without departing from the spirit and scope of this invention. The amounts indicated for the liquid film layer relate to the composition used to form the liquid film layers prior to drying.

All exemplified film compositions can be prepared by conventional formulation and mixing techniques.

### Example I

The following is an example of a bi-layer, teeth whitening device of the present invention.

**Whitening Composition Layer**

| **INGREDIENT** | **AMOUNT (weight percent) Liquid Film Layer** |
|---|---|
| XANTHAN GUM¹ | 0.0174% w/w |
| LOCUST BEAN GUM, CLARIFIED ² | 0.0348% w/w |
| CARRAGEENAN³ | 0.1740% w/w |
| PULLULAN⁴ | 4.1000% w/w |
| PLASDONE, USP K-90⁵ | 12.4000% w/w |
| SUCRALOSE⁶ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁷ | 5.7100% w/w |
| FLAVOR | 2.5890% w/w |
| POLYSORBATE 80 NF/EP⁸ | 0.3550% w/w |
| EMULSIFIER⁹ | 0.3550% w/w |
| GLYCERIN USP SPECIAL | 1.0000% w/w |
| | |

| **Disintegrable Layer** | |
|---|---|
| PHARMACEUTICAL GLAZE, 4-LB CUT NF¹⁰ | 55.0000% w/w |
| SILICA¹¹ (fumed untreated) | 4.0000% w/w |
| ALCOHOL USP/EP | 40.0000% w/w |
| GLYCERYL STEARATE SE¹² | 1.0000% w/w |

| | |
|---|---|
| ¹ Supplied under the name Keltrol T by CP Kelco, Chicago, IL ² Sold under the name Viscogum BCR 20/80 by Degussa Texturant Systems, Atlanta, GA ³ Supplied under the name Viscarin SD339 by FMC Biopolymer, Philadelphia, PA. ⁴ PI-20 grade supplied by Hayashibara. ⁵ Polyvinylpyrrolidone, USP K-90, International Specialties Products (ISP), Wayne, NJ. ⁶ Supplied under the tradename Splenda®, by McNeil Pharmaceuticals, New Brunswick, NJ. ⁷ ALB CG 35% hydrogen peroxide solution, Atofina, Philadelphia, Pa. ⁸ Tween 80, supplied by Quest, Hoffmann Estates, III. ⁹ Mixture of mono- and di-oleates supplied under name Atmos 300 by American Ingredients, Kansas City, Mo. ¹⁰ 35.5% Shellac supplied by Mantrose Haeser Co., Attleboro, Ma. ¹¹ Supplied under the tradename Cabosil® by Cabot, Tuscola, III. ¹² Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), xanthan gum, locust bean gum, carrageenan, pullulan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and emulsifier are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick sheet or surface at room temperature and dried under warm air to form the disintegrable layer of the bi-layer, teeth whitening film.

The contents of beaker A is then cast at desired thickness over the disintegrable layer at room temperature and dried under warm air to form the second layer of the bi-layer, teeth whitening film.

### Example II

The following is an example of a bi-layer, teeth whitening device of the present invention.

**Whitening Composition Layer**

| **INGREDIENT** | **AMOUNT (weight percent) Liquid Film Layer** |
|---|---|
| XANTHAN GUM¹ | 0.02308% w/w |
| LOCUST BEAN GUM, CLARIFIED² | 0.04616% w/w |
| CARRAGEENAN³ | 0.2308% w/w |
| PLASDONE, USP K-90⁴ | 16.426% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSIFIER⁸ | 0.3550% w/w |
| GLYCERIN USP SPECIAL | 1.0000% w/w |
| | |

| **Disintegrable Layer** | |
|---|---|
| PHARMACEUTICAL GLAZE, 4-LB CUT NF⁹ | 55.0000% w/w |
| SILICA¹⁰ (fumed untreated) | 4.0000% w/w |
| ALCOHOL USP/EP | 40.0000% w/w |
| GLYCERYL STEARATE SE¹¹ | 1.0000% w/w |

| | |
|---|---|
| ¹Supplied under the name Keltrol T by CP Kelco, Chicago, IL ² Sold under the name Viscogum BCR 20/80 by Degussa Texturant Systems, Atlanta, GA ³ Supplied under the name Viscarin SD339 by FMC Biopolymer, Philadelphia, PA. ⁴ Polyvinylpyrrolidone, USP K-90, International Specialties Products (ISP), Wayne, NJ. ⁵ Supplied under the tradename Splenda®, by McNeil Pharmaceuticals, New Brunswick, NJ. ⁶ ALB CG 35% hydrogen peroxide solution, Atofina, Philadelphia, Pa. ⁷ Tween 80, supplied by Quest, Hoffmann Estates, III. ⁸ Mixture of mono- and di-oleates supplied under name Atmos 300 by American Ingredients, Kansas City, Mo. ⁹ 35.5% Shellac supplied by Mantrose Haeser Co., Attleboro, Ma. ¹⁰ Supplied under the tradename Cabosil® by Cabot, Tuscola, III. ¹¹ Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), xanthan gum, locust bean gum, carrageenan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and emulsifier are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick sheet or surface at room temperature and dried under warm air to form the disintegrable layer of the bi-layer, teeth whitening film.

The contents of beaker A is then cast at desired thickness over the disintegrable layer at room temperature and dried under warm air to form the second layer of the bi-layer, teeth whitening film.

### Example III

The following is an example of a bi-layer, teeth whitening device of the present invention.

**Whitening Composition Layer**

| **INGREDIENT** | **AMOUNT (weight percent) Liquid Film Layer** |
|---|---|
| XANTHAN GUM¹ | 0.0674% w/w |
| LOCUST BEAN GUM, CLARIFIED² | 0.0848% w/w |
| PULLULAN³ | 4.1740% w/w |
| PLASDONE, USP K-90⁴ | 12.4000% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSIFIER⁸ | 0.3550% w/w |
| GLYCERIN USP SPECIAL | 1.0000% w/w |
| | |

| **Disintegrable Layer** | |
|---|---|
| PHARMACEUTICAL GLAZE, 4-LB CUT NF⁹ | 55.0000% w/w |
| SILICA¹⁰ (fumed untreated) | 4.0000% w/w |
| ALCOHOL USP/EP | 40.0000% w/w |
| GLYCERYL STEARATE SE¹¹ | 1.0000% w/w |

| | |
|---|---|
| ¹Supplied under the name Keltrol T by CP Kelco, Chicago, IL ² Sold under the name Viscogum BCR 20/80 by Degussa Texturant Systems, Atlanta, GA ³ PI-20 grade supplied by Hayashibara. ⁴ Polyvinylpyrrolidone, USP K-90, International Specialties Products (ISP), Wayne, NJ. ⁵ Supplied under the tradename Splenda®, by McNeil Pharmaceuticals, New Brunswick, NJ. ⁶ ALB CG 35% hydrogen peroxide solution, Atofina, Philadelphia, Pa. ⁷ Tween 80, supplied by Quest, _Hoffmann Estates, II1. ⁸ mixture of mono- and di-oleates supplied under name Atmos 300 by American Ingredients, Kansas City, Mo. ⁹ 35.5% Shellac supplied by Mantrose Haeser Co., Attleboro, Ma. ¹⁰ Supplied under the tradename Cabosil® by Cabot, Tuscola, III. ¹¹Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), xanthan gum, locust bean gum, pullulan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and emulsifier are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick sheet or surface at room temperature and dried under warm air to form the disintegrable layer of the bi-layer, teeth whitening film.

The contents of beaker A is then cast at desired thickness over the disintegrable layer at room temperature and dried under warm air to form the second layer of the bi-layer, teeth whitening film.

### Example IV

The following is an example of a bi-layer, teeth whitening device of the present invention.

**Whitening Composition Layer**

| **INGREDIENT** | **AMOUNT (weight percent) Liquid Film Layer** |
|---|---|
| STARCH GUM¹ | 1.9674% w/w |
| GUM ARABIC² | 0.1848% w/w |
| PULLULAN³ | 2.1740% w/w |
| PLASDONE, USP K-90⁴ | 12.4000% w/w |
| SUCRALOSE⁵ | 0.7000% w/w |
| POTASSIUM PHOSPHATE MONOBASIC NF | 0.0700% w/w |
| PURIFIED WATER, USP/EP | 72.4948% w/w |
| HYDROGEN PEROXIDE 35%⁶ | 5.7100% w/w |
| FLAVOR | 2.5890% w/w |
| POLYSORBATE 80 NF/EP⁷ | 0.3550% w/w |
| EMULSIFIER⁸ | 0.3550% w/w |
| GLYCERIN USP SPECIAL | 1.0000% w/w |

| **Disintegrable Layer** | |
|---|---|
| PHARMACEUTICAL GLAZE, 4-LB CUT NF⁹ | 55.0000% w/w |
| SILICA¹⁰ (fumed untreated) | 4.0000% w/w |
| ALCOHOL USP/EP | 40.0000% w/w |
| GLYCERYL STEARATE SE¹¹ | 1.0000% w/w |

| | |
|---|---|
| ¹ Supplied under the trade name of Pure-Cote B760, supplied by Grain processing Corporation, Muscatine, IA. ²Supplied under the name Bright Gum Arabic Spray Dry FCC/NF Powder by TIC Gums, Belcamp, MD ³ PI-20 grade supplied by Hayashibara. ⁴ Polyvinylpyrrolidone, USP K-90, International Specialties Products (ISP), Wayne, NJ. ⁵Supplied under the tradename Splenda®, by McNeil Pharmaceuticals, New Brunswick, NJ. ⁶ ALB CG 35% hydrogen peroxide solution, Atofina, Philadelphia, Pa. ⁷ Tween 80, supplied by Quest, Hoffmann Estates, Ill. ⁸ mixture of mono- and di-oleates supplied under name Atmos 300 by American Ingredients, Kansas City, Mo. ⁹ 35.5% Shellac supplied by Mantrose Haeser Co., Attleboro, Ma. ¹⁰ Supplied under the tradename Cabosil® by Cabot, Tuscola, III. ¹¹ Supplied as Mono- and Diglycerides of fats and oils (disposable grade) by Lonza Inc., Fair Lawn, NJ. | |

In a suitable beaker (beaker A), water, sucralose, potassium phosphate monobasic are added with mixing until the mixture is homogenous.

In a separate beaker (beaker B), starch gum, gum arabic, pullulan and Plasdone K-90 are mixed as a dry mix until the mixture is homogenous. The contents of beaker B are mixed into beaker A with rapid mixing or stirring. The combined mixture is mixed until the gums are hydrated. To the combined mixture, the hydrogen peroxide is added slowly with mixing.

In a separate beaker (beaker C), the flavor, polysorbate 80, glycerin and emulsifier are mixed until dissolved and uniform. The contents of beaker C are then poured into beaker A and mixed until the mixture is uniform and homogenous. The pH is then adjusted to about 5.5 using 1.0 N sodium hydroxide.

In still another separate beaker (beaker D), the pharmaceutical glaze, Cabosil, alcohol and glyceryl sterate is mixed until uniform and homogenous.

The contents of beaker D is then cast at desired thickness on a non-stick sheet or surface at room temperature and dried under warm air to form the disintegrable layer of the bi-layer, teeth whitening film.

The contents of beaker A is then cast at desired thickness over the disintegrable layer at room temperature and dried under warm air to form the second layer of the bi-layer, teeth whitening film.

### Example V

The following is an example of a bi-layer, teeth whitening device of the present invention.

**Whitening Composition Layer**

| **INGREDIENT** | **AMOUNT (weight percent) Liquid Film Layer** | **AMOUNT (weight percent) Dry Film Layer** |
|---|---|---|
| XANTHAN GUM¹ | 0.0200% w/w | 0.0695% w/w |
| LOCUST BEAN GUM, CLARIFIED² | 0.0400% w/w | 0.1389% w/w |
| CARRAGEENAN³ | 0.200% w/w | 0.6945 w/w |
| PULLULAN | 3.00 | 10.4179 w/w |
| PLASDONE, USP K-90⁴ | 14.0000% w/w | 48.6167 w/w |
| SACCHARIN SODIUM USP | 0.5000% w/w | 1.7363 w/w |
| POLYETHYLENE GLYCOL (PEG) 3350 NF | 0.500 | 1.7363 w/w |
| POTASSIUM PHOSPHATE MONOBASIC NF | 0.2600% w/w | 0.9029 w/w |
| DIBASIC SODIUM PHOSPHATE | 0.0900% w/w | 0.3125 w/w |
| ANHYDROUS NF | | |
| PURIFIED WATER, USP/EP | 64.9726% w/w | 10.5931 w/w |
| HYDROGEN PEROXIDE 35%⁵ | 12.7174% w/w | 11.9328 w/w |
| FLAVOR | 2.5000% w/w | 8.6815 w/w |
| POLYSORBATE 20 NF/EP⁶ | 0.350% w/w | 1.2154 w/w |
| EMULSIFIER⁷ | 0.35000% w/w | 1.7363 w/w |
| GLYCERIN USP SPECIAL | 0.5000% w/w | 1.7363 w/w |
| | | |
| **Disintegrable Layer** | | |
| PHARMACEUTICAL GLAZE, 4-LB CUT NF⁸ | 54.8272% w/w | 67.1845 w/w |
| SILICA⁹ (fumed untreated) | 4.0143% w/w | 13.8565 w/w |
| ALCOHOL USP/EP | 35.6660% w/w | 0.0000 w/w |
| TRIACETIN¹⁰ | 0.2452% w/w | 0.8464 w/w |
| ATMOS 300" | 4.7495% w/w | 1.7183 w/w |
| CAPRYLIC CAPRIC TRIGLYCERIDE ¹² | 0.4978% w/w | 16.3943 w/w |

| | | |
|---|---|---|
| ¹Supplied under the name Keltrol T by CP Kelco, Chicago, IL ²Sold under the name Viscogum BCR 20/80 by Degussa Texturant Systems, Atlanta, GA ³Supplied under the name Viscarin SD339 by FMC Biopolymer, Philadelphia, PA. ⁴ Polyvinylpyrrolidone, USP K-90, International Specialties Products (ISP), Wayne, NJ. ⁵ALB CG 35% hydrogen peroxide solution, Atofina, Philadelphia, Pa. ⁶Tween 80, supplied by Quest, Hoffmann Estates, III. ⁷Mixture of mono- and di-oleates supplied under name Atmos 300 by American Ingredients, Kansas City, Mo. ⁸ 35.5% Shellac supplied by Mantrose Haeser Co., Attleboro, Ma. ⁹ Supplied under the tradename Cabosil® by Cabot, Tuscola, III. ¹⁰ Eastman Triacetin (food grade) supplied by Eastman Chemical Company, Kingsport, TN. ¹¹ Mixture of mono- and di-oleates supplied by American Ingredients, Kansas City, Mo. ¹² Supplied under the the tradename Neobee 1053 by Stepan, Chicago, IL | | |

In a suitable vessel (vessel A), water, saccharin sodium, potassium phosphate monobasic, sodium phosphate dibasic and PEG 3350 are added with mixing until the mixture is homogenous for 30 minutes.

Next, the hydrogen peroxide is added to vessel A slowly with mixing.

In a separate vessel (vessel B), xanthan gum, locust bean gum, carrageenan, pullulan and Plasdone K-90 are mixed as a dry mix until the mixture is homogenous. The contents of vessel B are mixed into vessel A with rapid mixing or stirring. The combined mixture is mixed until the gums are hydrated for 2 hours.

In a separate vessel (vessel C), the flavor, polysorbate 80, glycerin and emulsifier are mixed until dissolved and uniform. The contents of vessel C are then poured into vessel A and mixed until the mixture is uniform and homogenous. The pH is then adjusted, if needed, to about 5.7, if needed, using 1.0 N sodium hydroxide.

The mixture in vessel A is next homogenized for about 10 minutes using an homogenizer such as the fixed speed 5 gallon-5D12T kettle - Lee Industries, Philadelphia, Pa. (The timing and equipment used for homogenization will, of course, vary depending on the batch sizes - for example, a 800kg batch size requires homogenization for about 60 to about 75 minutes at speeds of about 800-1300rpms using a Symex CML 2000 homogenizing vessel). The mixture is then transferred to continuously stirring vessels.

In still another separate vessel (vessel D), the pharmaceutical glaze, silica, alcohol Triacetin, Atmos 300 and Caprylic Capric Triglyceride 1053 is mixed until uniform and homogenous.

The contents of vessel D is then cast at desired thickness on a non-stick polypropylene sheet at room temperature and dried under warm air to form the disintegrable layer of the bi-layer, teeth whitening film.

The contents of vessel A is then cast at desired thickness over the disintegrable layer at room temperature and dried under warm air to form the second layer of the bi-layer, teeth whitening film.

## Claims

1. A multi-layer device for whitening teeth, comprising
i.) a first layer comprising:
a.) at least one water insoluble polymer; and
b.) at least one disintegration facilitator selected from the group consisting of water insoluble particulates, plasticizers and mixtures thereof; and
ii.) a second layer comprising:
a.) at least one tooth whitening active; and
b.) at least one water soluble polymer
wherein the device is erodible in an aqueous environment.

2. A device according to Claim 1 wherein the water insoluble polymer is selected from the group consisting of hydrogenated caster oil, polyvinyl chloride, shellac, cellulose derivatives, wax or mixtures thereof.

3. A device according to Claim 2 wherein the water insoluble polymer is shellac.

4. A device according to Claim 3 wherein the shellac has a molecular weight of from about 300 to about 700 daltons.

5. A device according to any preceding claim wherein the plasticizer is selected from the group consisting of citric acid alkyl esters, glycerol esters, phthalic acid alkyl esters, sebacic acid alkyl esters, sucrose esters, sorbitan esters, acetylated monoglycerides, glycerols, glycols, fatty acid esters, propylene glycol, triglycerides of caprylic/capric acids, poloxamers, alkyl aryl phosphates and polyethylene glycols 200 to 12,000 and mixtures thereof.

6. A device according to Claim 5 wherein the plasticizer is triglycerides of caprylic/capric acids.

7. A device according to any preceding claim wherein the water insoluble particulate is selected from the group consisting of alumina, talc, titanium dioxide, magnesium stearate, barium titanate, magnesium titanate, calcium titanate, strontium titanate, zinc oxide, silica sand, clay, mica, tabular spar, diatomaceous earth, various inorganic oxide pigments, chromium oxide, cerium oxide, antimony trioxide, magnesium oxide, zirconium oxide, barium sulfate, barium carbonate, calcium carbonate, silica, fumed silica, silicon carbide, silicon nitride, boron carbide, titanium carbide, and mixtures thereof.

8. A device according to Claim 7, wherein the water insoluble polymer is fumed silica.

9. A device according to Claim 8, wherein the fumed silica is present at a concentration of from about 0.1 to about 20% by weight of the wet first layer composition.

10. A device according to any preceding claim, wherein the water soluble polymer is selected form the group consisting of polyvinyl alcohol, polyvinyl pyrrolidone, carrageenan, locust bean gum, guar gum, hydroxy ethyl cellulose, xanthan gum, tragacanth gum, pullulan, starch and mixtures thereof.

11. A device according to Claim 10, wherein the water soluble polymer is polyvinyl pyrrolidone.

12. A device according to Claim 11, wherein the polyvinyl pyrrolidone is present at a concentration of from about 0.1% to about 30% by weight of the wet water soluble layer.

13. A device according to Claim 11 or Claim 12, wherein the polyvinyl pyrrolidone has an average molecular weight of from about 3,000 to about 2,000,000 daltons.

14. A device according to any preceding claim, wherein the teeth whitening active is selected from the group consisting of oxalates, peroxides, metal chlorites, perborates, percarbonates, peroxyacids, and mixtures thereof.

15. A device according to Claim 14, wherein the teeth whitening active is a peroxide.

16. A device according to Claim 15, wherein the peroxide is selected from the group consisting of hydrogen peroxide, calcium peroxide, sodium peroxide, carbamide peroxide, urea peroxide, sodium percarbonate and mixtures thereof.

17. A device according to Claim 16, wherein the peroxide is hydrogen peroxide.

18. A device according to any preceding claim wherein the water permeability of the first layer increases upon hydration of the second layer.

19. A device according to any preceding claim wherein the first layer is disintegrable in an aqueous environment and the second layer is dissolvable or dispersible in the aqueous environment.

20. A multi-layer teeth whitening device, comprising
i.) a first layer comprising:
a.) at least one water insoluble polymer; and
b.) at least one disintegration facilitator selected from the group consisting of water insoluble particulates, plasticizers and mixtures thereof; and
ii.) a second layer comprising:
a.) at least one water soluble polymer; and
b.) at least one tooth whitening active
wherein the water permeability of the first layer increases upon hydration of the second layer.
